(19) Europäisches Patentamt
European Patent Office
Office européen des brevets

(11) Veröffentlichungsnummer: **0 406 839 B1**

# (12) EUROPÄISCHE PATENTSCHRIFT

(45) Veröffentlichungstag der Patentschrift: **01.02.95**

(51) Int. Cl.⁶: **C07D 309/06**, C07D 335/02

(21) Anmeldenummer: **90112786.0**

(22) Anmeldetag: **04.07.90**

## (54) Verfahren zur Herstellung von 1-(4-Tetrahydropyranyl)-oder 1-(4-Tetrahydrothiopyranyl)-prop-1-en-3-onen.

(30) Priorität: **04.07.89 JP 172424/89**

(43) Veröffentlichungstag der Anmeldung:
**09.01.91 Patentblatt 91/02**

(45) Bekanntmachung des Hinweises auf die Patenterteilung:
**01.02.95 Patentblatt 95/05**

(84) Benannte Vertragsstaaten:
**BE CH DE FR GB LI NL**

(56) Entgegenhaltungen:
**EP-A- 0 298 380**
**EP-A- 0 352 456**
**US-A- 4 548 932**

**HOUBEN-WEYL: "Methoden der Organischen Chemie", Band XIII/2a, 1973, Seiten 321-323, Georg Thieme, Stuttgart, DE; "Metallorganische Verbindungen"**

(73) Patentinhaber: **BASF Aktiengesellschaft**
**Carl-Bosch-Strasse 38**
**D-67063 Ludwigshafen (DE)**

(72) Erfinder: **Kozima, Shigeru**
**950 Ohaza Huzisawa,**
**Nakagoumura**
**Nakakubikigun,**
**Niigata (JP)**
Erfinder: **Hatano, Masami**
**345 Aza Yanagimachi,**
**Takade**
**Odwareshi,**
**Kanagawa (JP)**

**EP 0 406 839 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 1-(4-Tetrahydropyranyl)- oder 1-(4-Tetrahydrothiopyranyl)-pro-1-en-3-onen sowie neue 1-(4-Tetrahydropyranyl)- oder 1-(4-Tetrahydrothiopyranyl)-prop-1-en-3-one.

Aus der US-A-4 548 932 ist in Beispiel 120 die Verbindung Ethyl-3-(3,4,5,6-tetrahydro-2H-pyran-4-yl)-acrylat [1-(4-Tetrahydropyranyl)-eth-len-2-carbonsäure-ethylester] bekannt.

Aus der DE-A-34 37 238 und der DE-A-35 36 117 ist die Herstellung von $\alpha,\beta$-ungesättigten Carbonylverbindungen durch Kondensationsreaktion von Aldehyden mit Ketonen, speziell Aceton, unter Basenkatalyse bekannt. Hierbei werden $\alpha,\beta$- und $\beta,\gamma$-Isomerengemische erhalten, die nur unter erheblichem Aufwand getrennt werden können.

Der vorliegenden Erfindung lag daher die Aufgabe zugrunde, den zuvorgenannten Nachteilen abzuhelfen.

Demgemäß wird ein neues und verbessertes Verfahren zur Herstellung von 1-(4-Tetrahydropyranyl)- oder 1-(4-Tetrahydrothiopyranyl)-prop-1-en-3-onen der allgemeinen Formel I

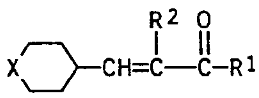 (I),

in der

- $R^1$ Wasserstoff, $C_1$- bis $C_{10}$-Alkyl, $C_1$- bis $C_{10}$-Alkoxy oder Aryloxy,
- $R^2$ Wasserstoff, $C_1$- bis $C_4$-Alkyl oder gegebenenfalls durch $C_1$- bis $C_4$-Alkyl und/oder Halogen substituiertes Aryl und
- X Sauerstoff oder Schwefel

bedeuten, gefunden, welches dadurch gekennzeichnet ist, daß man 1-Aminoprop-1-en-3-one der allgemeinen Formel II

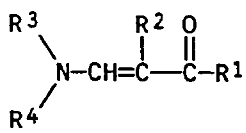 (II),

in der $R^1$ und $R^2$ die obengenannten Bedeutungen haben, und

- $R^3$, $R^4$ unabhängig voneinander Wasserstoff, $C_1$- bis $C_4$-Alkyl, Aryl oder gemeinsam eine $C_2$- bis $C_7$-Alkylenkette

bedeuten, mit einem 4-Tetrahydropyranyl- oder 4-Tetrahydrothiopyranylmagnesiumhalogenid der allgemeinen Formel III

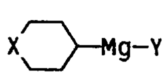 (III),

in der X die obengenannte Bedeutung hat und Y für Halogen steht, im Molverhältnis von III zu II von 0,8 : 1 bis 20 : 1 bei Temperaturen von (-20) bis 100 °C umsetzt, sowie neue 1-(4-Tetrahydropyranyl)- oder 1-(4-Tetrahydrothiopyranyl)-prop-1-en-3-onen der allgemeinen Formel I'

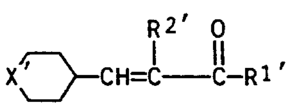 (I'),

in der die Substituenten folgende Bedeutung haben:

- $R^{1'}$ Wasserstoff, $C_1$- bis $C_{10}$-Alkyl oder Aryloxy,
- $R^{2'}$ Wasserstoff, $C_1$- bis $C_4$-Alkyl oder gegebenenfalls durch $C_1$- bis $C_4$-Alkyl und/oder Halogen

substituiertes Aryl und

X' Sauerstoff oder Schwefel.

Das erfindungsgemäpe Verfahren läßt sich wie folgt durchführen:

Zu einer Lösung bzw. Suspension oder Aufschlämmung eines 4-Tetrahydropyranyl- oder 4-Tetrahydrothiopyranylmagnesiumhalogenids der Formel III wird bei Temperaturen von (-20) bis 100 °C, vorzugsweise (-10) bis 50 °C, besonders bevorzugt bei 0 bis 20 °C ein 1-Aminoprop-1-en-3-on der Formel II, zugegeben.

Das Molverhältnis von III zu II beträgt zwischen 0,8:1 und 20:1, bevorzugt 1:1 bis 5:1, besonders bevorzugt 1:1 bis 1,2:1.

Als Lösungsmittel eignen sich alle für Grignardreaktionen üblichen Lösungsmittel beispielsweise dipolare, aprotische organische Lösungsmittel, z.B. Ether, wie Diethylether, Methyl-tert.-butylether, Tetrahydrofuran, Dioxan und Dimethylglykolether; oder Amine, wie Triethylamin, oder apolare, aprotische organische Lösungsmittel, z.B. aliphatische Kohlenwasserstoffe, wie Cyclohexan oder aromatische Kohlenwasserstoffe, wie Benzol, Toluol als Gemisch mit dipolaren, aprotischen Lösungsmitteln. Bevorzugt werden acyclische und cyclische Ether, wie Diethylether, Methyl-tert.-butylether, Tetrahydrofuran, Dioxan und Dimethylglykolether. Besonders bevorzugt werden cyclische Ether, wie Tetrahydrofuran und Dioxan.

Als Tetrahydropyranyl- und Tetrahydrothiopyranylmagnesiumhalogenide der Formel III eignen sich die Fluoride, Chloride, Bromide und Jodide, bevorzugt werden Chloride und Bromide.

Die Tetrahydropyranyl- und Tetrahydrothiopyranylmagnesiumhalogenide der Formel III lassen sich wie üblich durch Umsetzung der Tetrahydropyranyl- und Tetrahydrothiopyranylhalogenide mit Magnesium in einem der für Grignardreaktionen üblichen Lösungsmitteln herstellen.

Die Tetrahydropyranyl- und Tetrahydrothiopyranylhalogenide sind aus Tetrahydropyran-4-ol und Tetrahydrothiopyran-4-ol leicht zugänglich.

Die Substituenten $R^1$, $R^2$ in den Verbindungen I und II, das cyclische Glied X in Verbindung I und $R^3$, $R^4$ in Verbindung II haben folgende Bedeutungen:

$R^1$

- Wasserstoff,
- $C_1$- bis $C_{10}$-Alkyl, bevorzugt $C_1$- bis $C_8$-Alkyl, besonders bevorzugt $C_1$- bis $C_4$-Alkyl, wie Methyl, Ethyl, n-Propyl, iso-Propyl, n-Butyl, iso-Butyl, sec.-Butyl und tert.-Butyl,
- $C_1$- bis $C_{10}$-Alkoxy, bevorzugt $C_1$- bis $C_8$-Alkoxy, besonders bevorzugt $C_1$- bis $C_4$-Alkoxy wie Methoxy, Ethoxy, n-Propoxy, iso-Propoxy, n-Butoxy, iso-Butoxy, sec.-Butoxy und tert.-Butoxy,
- Aryloxy, bevorzugt Phenoxy,

$R^2$

- Wasserstoff,
- $C_1$- bis $C_{10}$-Alkyl, bevorzugt $C_1$- bis $C_8$-Alkyl, besonders bevorzugt $C_1$- bis $C_4$-Alkyl, wie Methyl, Ethyl n-Propyl, iso-Propyl, n-Butyl, iso-Butyl, sec.-Butyl und tert.-Butyl,
- Aryl wie Phenyl, 1-Naphthyl und 2-Naphthyl, bevorzugt Phenyl,
- ein- bis dreifach durch $C_1$- bis $C_4$-Alkyl substituiertes Aryl, bevorzugt ein- bis dreifach durch $C_1$- bis $C_4$-Alkyl substituiertes Phenyl wie 2-Methylphenyl, 4-Methylphenyl, 2,4-Dimethylphenyl und 2-Methyl-4-isopropylphenyl,
- ein- bis dreifach durch Halogen substituiertes Aryl, bevorzugt ein- bis dreifach durch Halogen substituiertes Phenyl wie 2-Chlorphenyl, 2-Fluorphenyl, 3-Bromphenyl, 3-Chlorphenyl, 3-Bromphenyl, 3-Fluorphenyl, 4-Chlorphenyl, 4-Bromphenyl, 4-Fluorphenyl, 2,4-Dichlorphenyl und 2-Chlor-4-bromphenyl,
- ein- bis dreifach durch $C_1$- bis $C_4$-Alkyl und Halogen substituiertes Aryl, bevorzugt ein- bis dreifach durch $C_1$- bis $C_4$-Alkyl und Halogen substituiertes Phenyl 2-Chlor-4-Methylphenyl, 4-Chlor-2-methylphenyl, 4-Chlor-2-isopropylphenyl und 2-Fluor-4-methylphenyl.

X

- Sauerstoff,
- Schwefel,

$R^3$, $R^4$

- unabhängig voneinander
- Wasserstoff,
- $C_1$- bis $C_4$-Alkyl wie Methyl, Ethyl, n-Propyl, iso-Propyl, n-Butyl, iso-Butyl, sec.-Butyl und tert.-Butyl, bevorzugt Methyl und Ethyl,
- Aryl, wie Phenyl, 1-Naphthyl und 2-Naphthyl, bevorzugt Phenyl,

- oder gemeinsam eine $C_2$- bis $C_7$-Alkylendikette, wie $(CH_2)_2$, $(CH_2)_3$, $(CH_2)_4$, $(CH_2)_5$, $(CH_2)_6$ und $(CH_2)_7$, bevorzugt $(CH_2)_4$ und $(CH_2)_5$.

In den erfindungsgemäßen Verbindungen I' haben die Substituenten $R^{1'}$, $R^{2'}$ und das cyclische Glied X' folgende Bedeutungen:

$R^{1'}$

- Wasserstoff,
- $C_1$- bis $C_{10}$-Alkyl, bevorzugt $C_1$- bis $C_8$-Alkyl, besonders bevorzugt $C_1$- bis $C_4$-Alkyl, wie Methyl, Ethyl, n-Propyl, iso-Propyl, n-Butyl, iso-Butyl, sec.-Butyl und tert.-Butyl, -Aryloxy, bevorzugt Phenoxy,

$R^{2'}$

- Wasserstoff,
- $C_1$- bis $C_{10}$-Alkyl, bevorzugt $C_1$- bis $C_8$-Alkyl, besonders bevorzugt $C_1$- bis $C_4$-Alkyl, wie Methyl, Ethyl, n-Propyl, iso-Propyl, n-Butyl, iso-Butyl, sec.-Butyl und tert.-Butyl,
- Aryl wie Phenyl, 1-Naphthyl und 2-Naphthyl, bevorzugt Phenyl,
- ein- bis dreifach durch $C_1$- bis $C_4$-Alkyl substituiertes Aryl, bevorzugt ein- bis dreifach durch $C_1$- bis $C_4$-Alkyl substituiertes Phenyl wie 2-Methylphenyl, 4-Methylphenyl, 2,4-Dimethylphenyl und 2-Methyl-4-isopropylpenyl,
- ein- bis dreifach durch Halogen substituiertes Aryl, bevorzugt ein- bis dreifach durch Halogen substituiertes Phenyl wie 2-Chlorphenyl, 2-Fluorphenyl, 3-Bromphenyl, 3-Chlorphenyl, 3-Bromphenyl, 3-Fluorphenyl, 4-Chlorphenyl, 4-Bromphenyl, 4-Fluorphenyl, 2,4-Dichlorphenyl und 2-Chlor-4-bromphenyl,
- ein- bis dreifach durch $C_1$- bis $C_4$-Alkyl und Halogen substituiertes Aryl, bevorzugt ein- bis dreifach durch $C_1$- bis $C_4$-Alkyl und Halogen substituiertes Phenyl wie 2-Chlor-4-methylphenyl, 2-Methyl-4-chlorphenyl, 4-Chlor-2-isopropylphenyl und 2-Fluor-4-methylphenyl.

X'

- Sauerstoff,
- Schwefel.

Unter den neuen Verbindungen I' seien beispielsweise genannt:

1-(4-Tetrahydropyranyl)-prop-1-en-3-on
1-(4-Tetrahydropyranyl)-but-1-en-3-on
1-(4-Tetrahydropyranyl)-pent-1-en-3-on
1-(4-Tetrahydropyranyl)-hex-1-en-3-on
1-(4-Tetrahydropyranyl)-hept-1-en-3-on
1-(4-Tetrahydropyranyl)-oct-1-en-3-on
1-(4-Tetrahydropyranyl)-non-1-en-3-on
1-(4-Tetrahydropyranyl)-dec-1-en-3-on
1-(4-Tetrahydropyranyl)-eth-1-en-2-carbonsäure-methylester
1-(4-Tetrahydropyranyl)-eth-1-en-2-carbonsäure-ethylester
1-(4-Tetrahydropyranyl)-eth-1-en-2-carbonsäure-n-propylester
1-(4-Tetrahydropyranyl)-eth-1-en-2-carbonsäure-iso-propylester
1-(4-Tetrahydropyranyl)-eth-1-en-2-carbonsäure-n-butylester
1-(4-Tetrahydropyranyl)-eth-1-en-2-carbonsäure-iso-butylester
1-(4-Tetrahydropyranyl)-eth-1-en-2-carbonsäure-sec.-butylester
1-(4-Tetrahydropyranyl)-eth-1-en-2-carbonsäure-tert.-butylester
1-(4-Tetrahydropyranyl)-eth-1-en-2-carbonsäure-tert.-phenylester
1-(4-Tetrahydrothiopyranyl)-prop-1-en-3-on
1-(4-Tetrahydrothiopyranyl)-but-1-en-3-on
1-(4-Tetrahydrothiopyranyl)-pent-1-en-3-on
1-(4-Tetrahydrothiopyranyl)-hex-1-en-3-on
1-(4-Tetrahydrothiopyranyl)-hept-1-en-3-on
1-(4-Tetrahydrothiopyranyl)-oct-1-en-3-on
1-(4-Tetrahydrothiopyranyl)-non-1-en-3-on
1-(4-Tetrahydrothiopyranyl)-dec-1-en-3-on
1-(4-Tetrahydrothiopyranyl)-eth-1-en-2-carbonsäure-methylester
1-(4-Tetrahydrothiopyranyl)-eth-1-en-2-carbonsäure-ethylester
1-(4-Tetrahydrothiopyranyl)-eth-1-en-2-carbonsäure-n-propylester
1-(4-Tetrahydrothiopyranyl)-eth-1-en-2-carbonsäure-iso-propylester

1-(4-Tetrahydrothiopyranyl)-eth-1-en-2-carbonsäure-n-butylester
1-(4-Tetrahydrothiopyranyl)-eth-1-en-2-carbonsäure-iso-butylester
1-(4-Tetrahydrothiopyranyl)-eth-1-en-2-carbonsäure-sec.-butylester
1-(4-Tetrahydrothiopyranyl)-eth-1-en-2-carbonsäure-tert.-butylester
1-(4-Tetrahydrothiopyranyl)-eth-1-en-2-carbonsäure-tert.-phenylester
Die gemäß dem erfindungsgemäßen Verfahren herstellbaren Verbindungen I sowie die neuen Verbindungen I' eignen sich als Zwischenprodukte für Wirkstoffe im Pflanzenschutz (DE-A-34 37 238, DE-A-35 36 117 und DE-A-36 01 066).

## Beispiele

### Beispiel 1

Herstellung von 1-(4-Tetrahydropyranyl)-but-1-en-3-on

5,0 g Magnesium wurden in 100 ml wasserfreiem Tetrahydrofuran vorgelegt. Unter Stickstoffatmosphäre wurden 25 g 4-Chlortetrahydropyran unter Rückfluß Zugetropft und 2 Std. am Rückfluß belassen. Anschließend wird zur auf 0 bis 5°C abgekühlten Reaktionsmischung eine Lösung von 23,5 g 1-(Dimethylamino)-but-1-en-3-on in 20 ml wasserfreiem Tetrahydrofuran so zugetropft, daß die Innentemperatur 50°C nicht überschreitet und 2 Std. bei Raumtemperatur nachgerührt. Zur Aufarbeitung gießt man die Reaktionsmischung auf Mischung aus Eis mit verdünntem HCl, extrahiert 3 mal mit 200 ml Chloroform, wäscht mit gesättigter Kochsalzlösung und trocknet über $MgSO_4$. Nach Entfernung des Lösungsmittels erhält man 24,0 g (75 %) 1-(4-Tetrahydropyranyl)-but-1-en-3-on mit einem Sdp. 117°C, 15 mbar (10 Torr).

### Beispiele 2 bis 6

Analog Beispiel 1 wurden die nachfolgend aufgeführten Beispiele durchgeführt. Die Ergebnisse sind in der folgenden Tabelle zusammengestellt.

| Beispiel Nr. | Lösungsmittel | 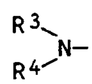 | Ausbeute |
|---|---|---|---|
| 2 | Diethylether | 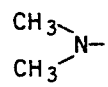 | 62 |
| 3 | Tetrahydrofuran | 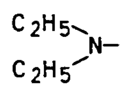 | 63 |
| 4 | Tetrahydrofuran | 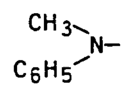 | 45 |
| 5 | Tetrahydrofuran | 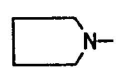 | |
| 6 | Tetrahydrofuran | 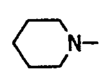 | |

### Beispiel 7

Herstellung von 1-(4-Tetrahydropyranyl)-1-buten-3-on

10,8 g (400 mmol) Mg-Späne werden in 30 ml Tetrahydrofuran mit einer katalytischen Menge Jod vorgelegt und mit 5 ml 4-Chlor-tetrahydropyran versetzt. Man erwärmt auf 50°C und tropft 45,2 g (375 mmol) 4-Chlor-tetrahydropyran, gelöst in 120 ml Tetrahydrofuran so zu, daß die Innentemperatur 70°C nicht übersteigt. Man rührt 1 Std. nach und kühlt anschließend auf Raumtemperatur ab. Unter Rühren tropft

man 42,4 g (375 mmol) 1-(Dimethylamino)-but-1-en-3-on zu, so daß die Reaktionstemperatur 30 °C nicht übersteigt. Anschließend rührt man 2 Std. nach, hydrolysiert und stellt die Lösung genau auf pH 7 ein. Die Phasen werden getrennt und die wäßrige Phase mehrmals mit 200 ml Essigsäureethylester oder Methyl-tert.-butylether extrahiert, getrocknet und fraktioniert destilliert. Man erhält 42,0 g (75 %) 1-(4-Tetrahydropyranyl)-but-1-en-3-on vom Sdp. 93 bis 95 °C; 10 mbar (5 Torr).

Beispiel 8

Herstellung von 1-(4-Tetrahydropyranyl)-eth-1-en-2-carbonsäureethylester

Man legt 9,6 g (400 mmol) Magnesium-Späne vor, überschichtet mit Tetrahydrofuran, gibt ca. 2 ml Brommethan zu und erwärmt zum Rückfluß. Man tropft 38,45 g (300 mmol) 4-Chlortetrahydropyran gelöst, in 100 ml Tetrahydrofuran zu. Anschließend rührt man noch 30 min nach, kühlt auf 10 °C, tropft 42,9 g (300 mmol) 1-(Dimethylamino)-eth-1-en-2-carbonsäureethylester zu und läßt 12 Std. nachrühren. Nach üblicher Aufarbeitung erhält man 31,4 g (60 %) 1-(4-Tetrahydropyranyl)-eth-1-en-2-carbonsäureethylester vom Sdp. 120 bis 140 °C; 15 mbar (10 Torr).

**Patentansprüche**

**1.** Verfahren zur Herstellung von 1-(4-Tetrahydropyranyl)- oder 1-(4-Tetrahydrothiopyranyl)-prop-1-en-3-onen der allgemeinen Formel I

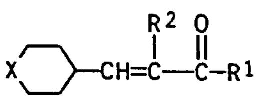

(I),

in der

$R^1$ Wasserstoff, $C_1$- bis $C_{10}$-Alkyl, $C_1$- bis $C_{10}$-Alkoxy oder Aryloxy,

$R^2$ Wasserstoff, $C_1$- bis $C_4$-Alkyl oder gegebenenfalls durch $C_1$- bis $C_4$-Alkyl und/oder Halogen substituiertes Aryl und

X Sauerstoff oder Schwefel

bedeuten, dadurch gekennzeichnet ist, daß man 1-Aminoprop-1-en-3-one der allgemeinen Formel II

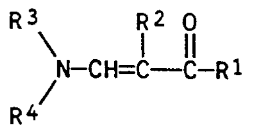

(II),

in der $R^1$ und $R^2$ die obengenannten Bedeutungen haben, und

$R^3$, $R^4$ unabhängig voneinander Wasserstoff, $C_1$- bis $C_4$-Alkyl, Aryl oder gemeinsam eine $C_2$- bis $C_7$-Alkylenkette

bedeuten, mit einem 4-Tetrahydropyranyl- oder 4-Tetrahydrothiopyranylmagnesiumhalogenid der allgemeinen Formel III

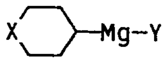

(III),

in der X die obengenannten Bedeutungen hat und Y für Halogen steht, im Molverhältnis von III zu II von 0,8:1 bis 20:1 bei Temperaturen von (-20) bis 100 °C umsetzt.

**2.** 1-(4-Tetrahydropyranyl)- oder 1-(4-Tetrahydrothiopyranyl)-prop-1-en-3-onen der allgemeinen Formel I'

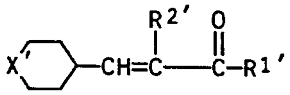 (I'),

in der die Substituenten folgende Bedeutung haben:

- $R^{1'}$ Wasserstoff, $C_1$- bis $C_{10}$-Alkyl oder Aryloxy,
- $R^{2'}$ Wasserstoff, $C_1$- bis $C_4$-Alkyl oder gegebenenfalls durch $C_1$- bis $C_4$-Alkyl und/oder Halogen substituiertes Aryl und
- X' Sauerstoff oder Schwefel.

## Claims

**1.** A process for the preparation of a 1-(4-tetrahydropyranyl)-or1-(4-tetrahydrothiopyranyl)prop-1-en-3-one of the general formula I

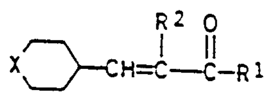 (I),

where

- $R^1$ is hydrogen, $C_1$- to $C_{10}$-alkyl, $C_1$- to $C_{10}$-alkoxy or aryloxy,
- $R^2$ is hydrogen, $C_1$- to $C_4$-alkyl, or aryl which is unsubstituted or substituted by $C_1$- to $C_4$-alkyl and/or halogen, and
- X is oxygen or sulfur,

which comprises reacting a 1-aminoprop-1-en-3-one of the general formula II

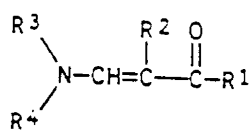 (II),

where $R^1$ and $R^2$ are as defined above, and

- $R^3$ and $R^4$, independently of one another, are hydrogen, $C_1$-to $C_4$-alkyl, aryl or together are a $C_2$- to $C_7$-alkylene chain,

with a 4-tetrahydropyranyl- or 4-tetrahydrothiopyranylmagnesium halide of the general formula III

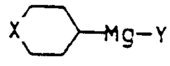 (III),

where X is as defined above and Y is halogen, in a molar ratio of III to II of from 0.8:1 to 20:1 at from -20 to 100 °C.

**2.** A 1-(4-tetrahydropyranyl)- or 1-(4-tetrahydrothiopyranyl)prop-1-en-3-one of the general formula I'

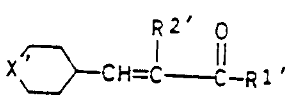 (I'),

where the substituents have the following meanings:

- $R^{1'}$ is hydrogen, $C_1$- to $C_{10}$-alkyl or aryloxy,
- $R^{2'}$ is hydrogen, $C_1$- to $C_4$-alkyl, or aryl which is unsubstituted or substituted by $C_1$- to $C_4$-alkyl and/or halogen, and
- X' is oxygen or sulfur.

**Revendications**

**1.** Procédé de préparation de 1-(4-tétrahydropyrannyl)- ou 1-(4-tétrahydrothiopyrannyl)-prop-1-ène-3-ones de la formule générale I :

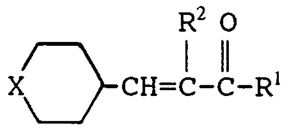

(I)

dans laquelle

$R^1$ représente un atome d'hydrogène, un radical alkyle en $C_1$-$C_{10}$, alcoxy en $C_1$-$C_{10}$, ou aryloxy;

$R^2$ représente un atome d'hydrogène, un radical alkyle en $C_1$-$C_4$, ou un radical aryle éventuellement substitué par des radicaux alkyle en $C_1$-$C_4$ et/ou des atomes d'halogène, et

X représente un atome d'oxygène ou de soufre,

caractérisé en ce que l'on fait réagir des 1-aminoprop-1-ène-3-ones de la formule générale II :

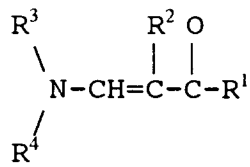

(II)

dans laquelle

$R^1$ et $R^2$ possèdent les significations qui leur ont été attribuées ci-dessus, et

$R^3$ et $R^4$ représentent, indépendamment l'un de l'autre, chacun un atome d'hydrogène, un radical alkyle en $C_1$-$C_4$, un radical aryle, ou forment ensemble une chaîne alkylène en $C_2$-$C_7$,

avec un halogénure de 4-tétrahydropyrannyl- ou 4-tétrahydrothiopyrannylmagnésium de la formule générale III :

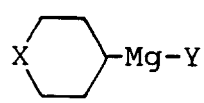

(III)

dans laquelle

X possède les significations qui lui ont été précédemment attribuées et Y représente un atome d'halogène,

dans le rapport de III à II de 0,8:1 à 20:1 et à des températures de -20 °C à 100 °C.

**2.** 1-(4-Tétrahydropyrannyl)- ou 1-(4-tétrahydrothiopyrannyl)-prop-1-ène-3-ones de la formule générale I' :

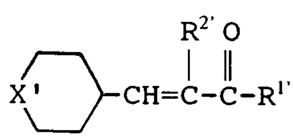

(I')

dans laquelle

$R^{1'}$ représente un atome d'hydrogène, un radical alkyle en $C_1$-$C_{10}$, ou un radical aryloxy;

$R^{2'}$ représente un atome d'hydrogène, un radical alkyle en $C_1$-$C_4$, ou un radical aryle éventuellement substitué par des radicaux alkyle en $C_1$-$C_4$ et/ou des atomes d'halogène, et

X' représente un atome d'oxygène ou de soufre.